# EUROPEAN PATENT SPECIFICATION

(11) **EP 0 571 911 B1**
(45) Date of publication and mention of the grant of the patent: **05.03.1997**
(21) Application number: 93108325.7
(22) Date of filing: 24.05.1993
(51) Int. Cl.: C12Q 1/68, C07H 21/04, C12P 19/34

(54) **Mycobacteria probes**
Sonden für Mycobakterien
Sondes pour mycobactéries

(30) Priority: 26.05.1992 US 889651
(43) Date of publication of application: 01.12.1993
(73) Proprietor: Becton Dickinson and Company, Franklin Lakes, New Jersey 07417-1880 (US)
(72) Inventor: Spears, Patricia A., Raleigh, North Carolina 27615 (US); Shank, Daryl D., Durham, North Carolina 27712 (US)
(74) Representative: Rambelli, Paolo

(56) References cited:
- WO-A-90/10085
- ABSTRACTS OF THE GENERAL MEETING OF THE AMERICAN SOCIETY FOR MICROBIOLOGY 26 May 1992 , WASHINGTON US page 166 P.A.SPEARS ET AL. 'The characterization of novel sequences for the identification of Mycobacteria by DNA hybridization and polymerase chain reaction amplification'

## Description

### Field of the invention

The present invention relates to oligonucleotide probes and particularly relates to oligonucleotide probes capable of selectively hybridising to Mycobacteria DNA.

### Background of the invention

Health care providers are encountering a significant increase in cases of mycobacterial infections. Many of these new cases are related to the AIDS epidemic. Physicians rely on clinical microbiologists to assist them in diagnosing a mycobacterial infection. The diagnosis of such infections is, however, largely dependent on acid-fast staining and cultivation of the organism, followed by biochemical assays. These are time consuming processes. WO90/10085 (Cogent Limited) describes probes for detection of mycobacteria and diagnosis of tuberculosis. The probes are derived from a repetitive element belonging to the IS3 family of insertion sequences. They hybridise to and distinguish between *M. tuberculosis, M. bovis* and BCG, but do not hybridise significantly to mycobacteria of other species. There is a continuing need for new, and particularly rapid, methods of diagnosing mycobacterial infections.

### Summary of the invention

The present invention provides an oligonucleotide probe capable of selectively hybridising to Mycobacteria nucleic acid. The oligonucleotide probe is selected from the group consisting of: *(a)* an oligonucleotide probe consisting essentially of the DNA sequence given herein as SEQ ID NO:1 (MK14); *(b)* oligonucleotide probes which hybridise to MK14 are at least 10 nucleotides in length and are at least 60% homologous with MK14 nucleic acid; and *(c)* oligonucleotide probes which are complementary to any of the foregoing and are capable of selectively hybridising to Mycobacteria nucleic acid.

A first embodiment of the foregoing is an oligonucleotide probe selected from the group consisting of: *(a)* an oligonucleotide probe consisting of the DNA sequence given herein as SEQ ID NO:22 (*M. kansasii* fragment BC), an oligonucleotide probe consisting of the DNA sequence given herein as SEQ ID NO:23 (*M. tuberculosis* fragment BC), and an oligonucleotide probe consisting of the sequence given herein as SEQ ID NO:24 (*M. avium* fragment BC); *(b)* oligonucleotide probes which hybridise to probes of (a) above are at least 10 nucleotides in length and are at least 60% homologous with MK14 nucleic acid; and *(c)* oligonucleotide probes which are complementary to any of the foregoing and are capable of selectively hybridising to Mycobacteria nucleic acid.

A second embodiment of the foregoing is an oligonucleotide probe selected from the group consisting of: *(a)* an oligonucleotide probe consisting of the DNA sequence given herein as SEQ ID NO:14 (MK14-C); *(b)* oligonucleotide probes which hybridise to MK14 are at least 10 nucleotides in length and are at least 60% homologous with MK14 nucleic acid; and (c) oligonucleotide probes which are complementary to any of the foregoing and are capable of selectively hybridising to Mycohacteria nucleic acid.

A third embodiment of the foregoing is an oligonucleotide probe capable of selectively hybridising to *Mycobacteria kansasii* nucleic acid, the oligonucleotide probe selected from the group consisting of: *(a)* an oligonucleotide probe consisting of the DNA sequence given herein as SEQ ID NO:15 (MK14-D); *(b)* oligonucleotide probes which hybridise to MK14 and are capable of selectively hybridising to *Mycobacteria kansasii* nucleic acid; and *(c)* oligonucleotide probes which are complementary to any of the foregoing oligonucleotide probes and are capable of selectively hybridising to Mycobacteria nucleic acid.

A method of detecting Mycobacteria nucleic acid in a nucleic acid sample is also disclosed herein. The method comprises the steps of contacting an oligonucleotide probe to a nucleic acid sample under conditions permitting the hybridisation of said oligonucleotide probe to Mycobacteria nucleic acid, wherein the oligonucleotide probe is a probe as given above, and then detecting whether or not the oligonucleotide probe hybridises to the nucleic acid sample, the hybridisation of the oligonucleotide probe to the nucleic acid sample indicating that the nucleic acid sample contains Mycobacteria nucleic acid.

Also disclosed herein is a kit for detecting Mycobacteria nucleic acid in a nucleic acid sample. The kit comprises a hybridisation solution together with an oligonucleotide probe as given above. The oligonucleotide probe may be either lyophilised or carried in the hybridisation solution.

A method of amplifying a selected Mycobacteria nucleic acid sequence in a nucleic acid sample is also disclosed herein. The amplification method comprises contacting at least one pair of oligonucleotide probes to the nucleic acid sample under conditions permitting the hybridisation of each of the oligonucleotide probes to the selected Mycobacteria nucleic acid sequence, wherein said pair of probes together comprise an amplification pair, and wherein each of said oligonucleotide probes is selected from the group consisting of *(i)* oligonucleotide probes comprising fragments of SEQ ID NO:1 (MK14) which retain the capability of MK14 of selectively hybridising to Mycobacteria nucleic acid; and *(ii)* oligonucleotide probes which are complementary to any of the foregoing are at least 10 nucleotides in length and are at least 60% homologous with MK14 nucleic acid; and then amplifying the selected mycobacteria target sequence.

### Brief description of the drawings

Figure 1 shows a map of clone MK14. The insert of plasmid MK14 was subdivided into 5 subfragments A-E. Along the DNA are oligonucleotides (oligos) designated as boxes and labelled as per oligo list (table 1 below). Each fragment size and GC content is as noted.

Figure 2 shows a Southern blot of mycobacteria and non-mycobacteria which was probed with MK14-C labelled with ³²P. This banding pattern shows strong genus specificity with no cross-reactivity seen in any non-mycobacteria tested.

Figure 3 shows a Southern blot of mycobacteria and non-mycobacteria which was probed with MK14-D labelled with ³²P. This probe shows strong *M. kansasii* specificity and does not cross-react with any other organisms tested.

Figure 4 shows a Polymerase Chain Reaction (PCR) agarose gel. PCR amplification reactions, using oligos 14-3 (SEQ ID NO:2) and 14-10 (SEQ ID NO:9) as primers, were analysed by visualising on a 1% ethidium bromide stained agarose gel. The arrow points to a 242 bp product found in all the lanes with slow growing mycobacteria used as template. As a positive control the plasmid MK14 (pMK14) was used as a template. As a negative control water was added instead of template DNA.

**Figure 5** shows a Southern blot of the PCR gel in figure 4 probed with MK14-C labeled with ³²P. The arrow points to a 242 bp product present in all the slow growing mycobacteria and *M. gordonae.* It is absent in all the non-mycobacteria, and absent in mycobacteria *M. chelonae* and *M. fortuitum.* The positive and negative controls are the same as those in figure 4.

### Detailed Description of the Invention

The present invention advantageously provides both probes which bind to a variety of different Mycobacteria species and probes which bind to specific Mycobacteria species. The general probes are useful as an initial screen for Mycobacterial infection, and provide a rapid alternative to the culturing techniques currently employed as an initial screen, which require on the order of six weeks of culturing. Once a positive result on the initial screen is found, the species specific probes herein provide a rapid means to diagnose the particular infection.

Nucleotide sequences are presented herein by single strand only, in the 5' to 3' direction, from left to right. One letter nucleotide symbols used herein have their standard meaning in the art in accordance with the recommendations of the IUPAC-IUB Biochemical Nomenclature Commission.

The term "mycobacteria" as used herein has its conventional meaning in the art referring to acid-fast, non-motile, rod shaped bacteria. *See generally* B. Davis et al., Microbiology, 724-742 (3d Ed. 1980). By way of example, the mycobacteria include, but are not limited to, *Mycobacterium africanum, M. avium, M. bovis, M. bovis-BCG, M. chelonae, M. fortuitum, M. gordonae, M. intracellulare, M. kansasii, M. microti, M. scrofulaceum,* and *M. tuberculosis.*

The term "amplification pair," as used herein, refers to a pair of oligonucleotide probes of the present invention selected to be suitable for use together in amplifying a selected mycobacteria nucleic acid sequence by a process such as polymerase chain reaction, ligase chain reaction, or strand displacement amplification, as explained in greater detail below.

Nucleic acid (i.e., DNA or RNA) samples for practicing the present invention may be obtained from any suitable source. Typically, the nucleic acid sample will be obtained in the form of a sample of a biological fluid or biological tissue suspected of containing mycobacteria. Suitable biological fluids include, but are not limited to, sputum, bronchial washings, gastric washings (containing swallowed sputum), blood, milk, and lymph fluid. Suitable tissue samples include, but are not limited to, skin and soft tissue samples. As mycobacteria infect both human and animal species, the present invention is applicable to both human and veterinary diagnostic procedures, and samples may be collected from both human and animal species. For example, *M. bovis* causes tuberculosis in cattle and is transmissible to humans, and hence the present invention may be used to diagnosis infection in cattle and to test cattle milk for the presence of *M. bovis* which may be transmitted to humans. Another example is *M. avium* and *M. intracellulare,* which infect birds (e.g., chickens and pigeons) as well as swine, and hence the present invention may be used to detect such infections. Further, humans are susceptible to infection from a variety of mycobacteria, including, but not limited to, *M. tuberculosis, M. kansasii, M. avium, M. intracellulaire, M. scrofulaceum* and *M. fortuitum,* and the present invention may be used to detect such infections.

Oligonucleotide probes of the present invention may be of any suitable length, depending on the particular assay format employed. In general, the oligonucleotide probes are at least 10 to 15 nucleotides in length. For example, oligonucleotide probes used for detecting mycobacteria are preferably 15 to 20 nucleotides in length. The oligonucleotide probes may incorporate the elements of a strand displacement amplification probe, as explained in detail below. Nucleic acid sequences to which amplification pairs of oligonucleotide probes are directed are preferably 50 to 150 nucleotides in length.

With respect to nucleic acid sequences which hybridise to specific nucleic acid sequences disclosed herein, hybridisation may be carried out under conditions of reduced stringency, medium stringency or even stringent conditions (e.g. conditions represented by a wash stringency of .5x SSC and .1% SDS at a temperature of 20 or 30 degrees below the melting temperature of the probe, or even conditions represented by a wash stringency of .1x SSC and .1% SDS at a temperature of 10 degrees below the melting temperature of the DNA sequence to MK14 DNA) in a standard hybridisation assay. *See* J.Sambrook et al, Molecular Cloning, A Laboratory Manual (2nd Ed. 1989) (Cold Spring Harbor Laboratory)). In general, nucleic acid sequences which hybridise to the MK14 DNA disclosed herein will be at least 60% homologous, 65% homologous, 70% homologous, and even 75% homologous or more with the sequence of the MK14 DNA disclosed herein.

Probes of the invention can be utilized with naturally occurring sugar-phosphate backbones as well as modified backbones including phosphorothioates, dithionates, alkyl phosphonates and α-nucleotides. Modified sugar-phosphate backbones are generally illustrated by Miller and T'so, Ann.Reports Med.Chem., 23:295 (1988) and Moran et al., Nuc. Acids Res., 14:5019 (1987). Probes of the invention can be constructed of either ribonucleic acid (RNA) or deoxyribonucleic acid (DNA), with DNA preferred.

Use of probes in detection methods include Northern blots (RNA detection), Southern blots (DNA detection), Western blots (protein detection), and dot blots (DNA, RNA or protein). Other detection methods include kits containing probes on a dipstick setup and the like.

To detect hybrid molecules formed from using the probes of the invention, typically a detectable marker is added to one of the probes. Probes can be labelled by several methods. Probes can be radiolabelled and detected by autoradiography. Such labels for autoradiography include ³H, ¹²⁵I,³⁵S, ¹⁴C, and ³²P. Typically the choice of radioactive isotopes depends on research preferences involving ease of synthesis, stability, and half lives of the isotopes. Other detectable markers include ligands, fluorophores, chemiluminescent agents, electrochemical via sensors, time-resolved fluorescence, enzymes, and antibodies. For example, an antibody can be labelled with a ligand. Other detectable markers for use with probes of the invention include biotin, radionucleotides, enzyme inhibitors, co-enzymes, luciferins, paramagnetic metals, spin labels, and monoclonal antibodies. The choice of label dictates the manner in which the label is bound to the probe.

Radioactive nucleotides can be incorporated into probes of the invention by several means. Such means include nick translation of double-stranded probes, copying single-stranded M13 plasmids having specific inserts with the Klenow fragment of DNA polymerase I of E. coli or other such DNA polymerase in the presence of radioactive dNTP, transcribing cDNA from RNA templates using reverse transcriptase in the presence of radioactive dNTP, transcribing RNA from vectors containing strong promoters such as SP6 promoters or T7 promoters using SP6 or T7 RNA polymerase in the presence of radioactive rNTP, tailing the 3' ends of probes with radioactive nucleotides using terminal transferase, and by phosphorylation of the 5' ends of probes using gamma ³²P ATP and polynucleotide kinase.

Amplification of a selected, or target, nucleic acid sequence may be carried out by any suitable means.

*See generally* D.Kwoh, *Am.Biotechol.Lab.* **8**, 14-25 (1990). Examples of suitable amplification techniques include, but are not limited to, polymerase chain reaction, ligase chain reaction, strand displacement amplification, transcription-based amplification (see D.Kwoh et al., *Proc.Natl.Acad.Sci.* USA **86**, 1173-1177 (1989)), self-sustained sequence replication *(see* J.Guatelli et al., Proc.Natl.Acad.Sci. USA 87, 1874-1878 (1990)), and the Qβ replicase system (*see* P.Lizardi et al., *BioTechnology* **6**, 1197-1202 (1988)).

Polymerase chain reaction (PCR) is carried out in accordance with known techniques. *See, e.g.,* U.S. patents Nos.4 683 195; 4 683 202; 4 800 159; and 4 965 188. In general, PCR involves, first, treating a nucleic acid sample (e.g. in the presence of a heat stable DNA polymerase) with one oligonucleotide primer for each strand of the specific sequence to be detected under hybridising conditions so that an extension product of each primer is synthesised which is complementary to each nucleic acid strand, with the primers sufficiently complementary to each strand of the specific sequence to hybridise therewith so that the extension product synthesised from each primer, when it is separated from its complement, can serve as a template for synthesis of the extension product of the other primer, and then treating the sample under denaturing conditions to separate the primer extension products from their templates if the sequence or sequences to be detected are present. These steps are repeated cyclically until the desired degree of amplification is obtained. Detection of the amplified sequence may be carried out by adding to the reaction product an oligonucleotide probe capable of hybridising to the reaction product (e.g., an oligonucleotide probe of the present invention), the probe carrying a detectable label, and then detecting the label in accordance with known techniques.

Ligase chain reaction (LCR) is carried out in accordance with known techniques. *See, e.g.,* R. Weiss, *Science* **254**, 1292 (1991). In general, the reaction is carried out with two pairs of oligonucleotide probes: one pair binds to one strand of the sequence to be detected; the other pair binds to the other strand of the sequence to be detected. Each pair together completely overlaps the strand to which it corresponds. The reaction is carried out by, first, denaturing (e.g., separating) the strands of the sequence to be detected, then reacting the strands with the two pairs of oligonucleotide probes in the presence of a heat stable ligase so that each pair of oligonucleotide probes is ligated together, then separating the reaction product, and then cyclically repeating the process until the sequence has been amplified to the desired degree. Detection may then be carried out in like manner as described above with respect to PCR.

Strand displacement amplification (SDA) is also carried out in accordance with known techniques. *See* G. Walker et al., *Proc. Natl. Acad. Sci. USA* **89**, 392-396 (1992); G. Walker et al., *Nucleic Acids Res*. **20**, 1691-1696 (1992). SDA may be carried out with a single amplification primer or a pair of amplification primers, with exponential amplification being achieved with the latter. In general, SDA amplification primers comprise, in the 5' to 3' direction, a flanking sequence (the DNA sequence of which is noncritical), a restriction site for the restriction enzyme employed in the reaction, and an oligonucleotide sequence (e.g., an oligonucleotide probe of the present invention) which hybridizes to the target sequence to be amplified and/or detected. The flanking sequence, which simply serves to facilitate binding of the restriction enzyme to the recognition site, is preferably about 15 to 20 nucleotides in length; the restriction site is functional in the SDA reaction (i.e., phosphorothioate linkages incorporated into the primer strand do not inhibit subsequent nicking--a condition which may be satisfied through use of a nonpalindromic recognition site); the oligonucleotide probe portion is preferably about 13 to 15 nucleotides in length. SDA is carried out with a single amplification primer as follows: a restriction fragment (preferably about 50 to 100 nucleotides in length and preferably of low GC content) containing the sequence to be detected is prepared by digesting a DNA sample with one or more restriction enzymes, the SDA amplification primer is added to a reaction mixture containing the restriction fragment so that a duplex between the restriction fragment and the amplification primer is formed with a 5' overhang at each end, a restriction enzyme which binds to the restriction site on the amplification probe (*e.g., Hinc*II) is added to the reaction mixture, an exonuclease deficient DNA polymerase (*e.g.,* an exonuclease deficient form of *E. coli* DNA polymerase I, *see* V. Derbyshire, *Science* **240**, 199-201 (1988)) is added to the reaction mixture, and three dNTPs and one dNTP[αS], with the dNTP[αS] selected so that a phosphorothioate linkage is incorporated into the primer strand at the restriction site for the particular restriction enzyme employed (*e.g.*, dGTP, dCTP, dTTP, and dATP[αS] when the restriction enzyme is *Hinc*II) are added to the reaction mixture. The DNA polymerase extends the 3' ends of the duplex with the dNTPs to form a downstream complement of the target strand, the restriction enzyme nicks the restriction site on the amplification primer, and the DNA polymerase extends the 3' end of the amplification primer at the nick to displace the previously formed downstream complement of the target strand. The process is inherently repetitive because the restriction enzyme continuously nicks new complementary strands as they are formed from the restriction site, and the DNA polymerase continuously forms new complementary strands from the nicked restriction site. SDA can be carried out with a pair of primers on a double stranded target DNA sequence, with the second primer binding to the 5' end of the complementary strand, so that two sets of repetitive reactions are occuring simultaneously, with the process proceeding exponentially because the products of one set of reactions serve as a target for the amplification primer in the other set of reactions. In addition, the step of first digesting the DNA sample to form a restriction fragment can be eliminated by exploiting the strand displacing activity of the DNA polymerase and adding a pair of "bumper" primers which bind to the substrate at a flanking position 5' to the position at which each amplification primer binds. Each bumper primer extension product displaces the corresponding amplification primer extension product, and the two displaced, complementary, amplification primer extension products bind to one another to form a double-stranded DNA fragment which can then serve as a substrate for exponential SDA with that pair of SDA primers.

When SDA is employed, the oligonucleotide probes of the invention are preferably selected so that guanine plus cytosine content is low, preferably comprising less than 70% of the total nucleotide composition of the probe. Similarly, the target sequence should be of low GC content to avoid the formation of secondary structures.

A kit for detecting mycobacteria nucleic acid in a nucleic acid sample contains at least one probe of the present invention, and hybridization solution for enabling hybridization between the probes and the nucleic acid sample, with the probe either suspended in the solution or provided separately in lyophylized form. One example of a suitable hybridization solution is a solution comprised of 6x SSC (0.9M sodium chloride, 0.09 M sodium citrate, pH 7.0), 0.1M EDTA pH 8.0, 5x Denhardt's solution [0.1% (w/v) Ficoll Type 400, 0.1% (w/v) polyvinylpyrrolidone, 0.1% (w/v) bovine serum albumin], and 100 µg/ml sheared, denatured salmon sperm DNA, commercially available from Bethesda Research Laboratories, Gaithersburg, MD 20877 USA under Catalog No. 5565UA. *See also* T. Maniatis et al., Molecular Cloning: A Laboratory Manual, 387-388 (1982)(Cold Spring Harbor Laboratory). The components of the kit are packaged together in a common container (e.g., a container sealed with a frangible seal), the kit typically including an instruction sheet for carrying out a specific embodiment of the method of the present invention. Additional optional components of the kit, depending on the assay format to be employed, include a second probe of the invention suitable for use with the first probe for carrying out PCR as explained above (or, in the case of a kit for carrying out LCR, two pairs of probes of the present invention), one or more detection probes, and means for carrying out a detecting step (e.g., a probe of the invention labelled with a detectable marker and optionally an enzyme substrate when the detectable marker is an enzyme).

The present invention is explained in greater detail in the following Examples, in which "Kb" means kilobases, "bp" means base pairs, "ng" means nanograms, "pmoles" means picomoles, "µl" means microliters, "mM" means milliMolar, "µM" means microMolar, "DTT" means dithiothreitol, "Tm" means melting temperature, and temperatures are given in degrees Centigrade unless otherwise indicated. These Examples are for illustrative purposes only, and are not to be taken as limiting of the invention.

### EXAMPLE 1

### Strains and Genomic DNA Preparation

Mycobacteria used in the Examples described herein were *M. africanum* LCDC501, *M. avium* ATCC25291, *M. bovis* CDC4, *M. bovis-BCG* CDC34, *M. chelonae* TMC1543, *M. fortuitum* TMC1529*, M. gordonae* TMC1318, *M. intracellulare* ATCC13950, *M. kansasii* TMC1201, *M. microti* LCDC201, *M. scrofulaceum* CDC78, and *M. tuberculosis* ATCC27294. Non-mycobacteria used were *Bordetella pertussis* ATCC8467, *Candida albicans* ATCC44808, *Corynebacterium diphtheriae* ATCC11913, *Escherichia coli* ATCC11775, *Flavobacterium meningisepticum* ATCC13253, *Nocardia asteroides* ATCC3308, *Rhodococcus rhodochrous* ATCC13808, *Streptococcus pneumoniae* ATCC6303, Adenovirus Sigma D3390 (Advirus), Eukaryotic DNA McCoy cells (eukDNA). The slow growing mycobacteria are *M. africanum, M. avium*, *M. bovis, M. bovis-BCG, M. intracellulare, M. kansasii, M. microti, M. scrofulaceum* and *M. tuberculosis.* The fast growing mycobacteria are *M. chelonae, M. fortuitum,* and *M. gordonae*. All of the mycobacteria strains were cultured in BACTEC (trademark) vials then heat killed at 70°C for 4 hours. The genomic DNA was isolated in accordance with known techniques. *See* S.Visuvanathan et al., Simple Enzymatic Method for Isolation of DNA from Diverse Bacteria, *J.Microbiol.Meth.* **10**, 59-64 (1989). The non-mycobacteria strains were cultured in Luria broth and the genomic DNA was isolated by the CTAB mini-prep method. *See, e.g.,* F.Asubel et al., Current Protocols in Molecular Biology (1987).

### EXAMPLE 2

### M. kansasii Library

*M. kansasii* genomic DNA was cut with *Sau3AI* (GIBCO BRL). The fragment sizes ranged from 1.4 Kb to < 200 bp. The vector, BlueScript SK+ (trademark) (Stratagene), was digested with *BamHI* (GIBCO BRL) and then phosphatased using Alkaline Phosphatase from calf intestine (CIAP) (Boehringer Mannheim Biochemicals). 750 ng of insert was ligated to 20 ng of vector in 50 mM Tris-HCl pH 7.5, 7 mM MgCl₂, 1 mM DTT, 1 mM ATP and 1 Unit/10 µl T4 DNA Ligase (GIBCO BRL). The ligation was incubated at 15°C overnight then transformed into competent JM109 cells (Stratagene). 1000 independent isolates were obtained.

### EXAMPLE 3

### Identification and Sequencing of Clone MK14

From the 1000 clones generated in Example 2 above, 15 were chosen for screening genomic blot which consisted of various mycobacterial and non-mycobacterial DNA's. Of the 15 clones, MK14 (SEQ ID NO:1) was found to hybridize to all the mycobacteria and not cross hybridize to any non-mycobacteria tested.

MK14 was sequenced using a Sequenase version 2.0 kit (United States Biochemicals) with [α-³⁵S]-deoxyadenosine triphosphate (NEN-Dupont). MK14 was also sequenced using the Applied Biosystems 373A DNA Sequencer using the dye primer kit as suggested by the manufacturer.

### EXAMPLE 4

### Oligonucleotide Synthesis

Oligonucleotides (oligos) were synthesized on an Applied Biosystems 380B Synthesizer as suggested by the manufacturer. The oligos were deblocked at 50°C and then purified through an Oligonucleotide Purification Cartridge as suggested by the manufacturer. Names and sequences of oligonucleotides are listed in **Table 1**.

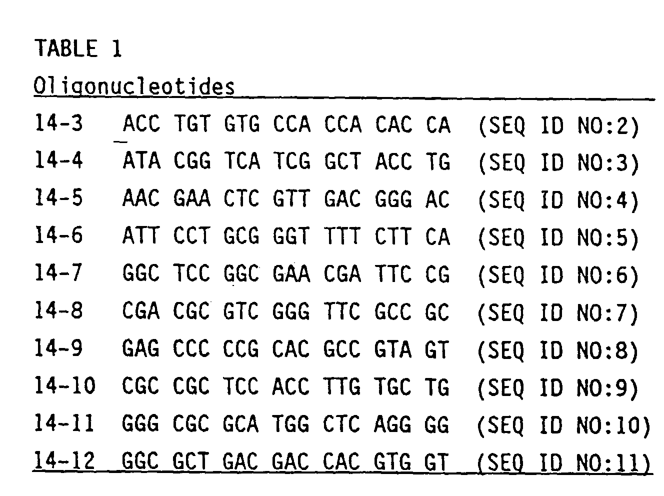

Note that, with respect to oligonucleotide 14-3 (SEQ ID NO:2), the T at the position corresponding to position 98 in MK14 (SEQ ID NO:1) is absent because the synthesis of this probe was based on earlier sequence information. While this change is incorporated into MK14 fragments synthesised with this oligonucleotide (discussed below), no adverse effect was seen.

### EXAMPLE 5

### Production of MK14 Fragments A to E by Polymerase Chain Reaction

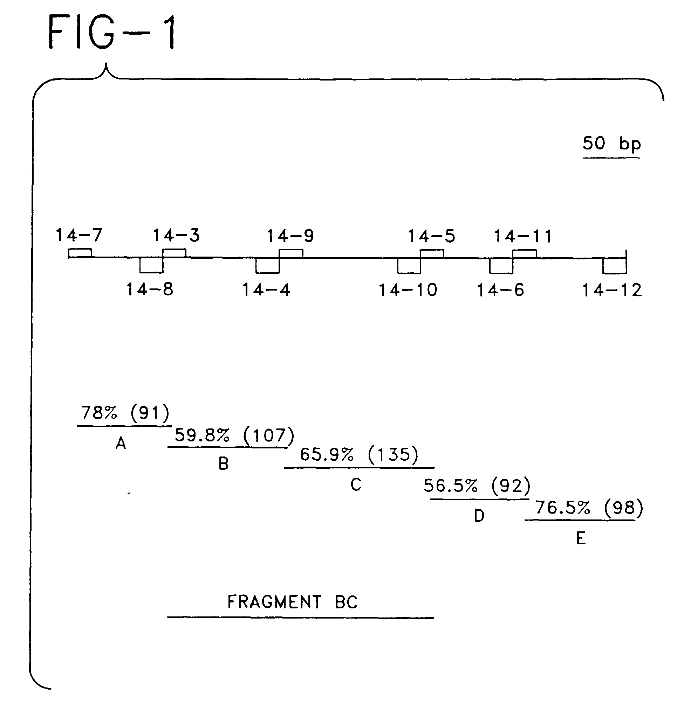

Clone MK14 was divided into five smaller fragments designated A to E by polymerase chain reaction using plasmid MK14 as the template and the oligonucleotides primers described in Example 4 above in the pattern shown in figure 1.

Polymerase chain reaction amplifications were done in 25-100 µl reactions consisting of 10 mM Tris-HCl pH 8.3, 50 mM KCl, 1.5 mM MgCl₂, .001% (w/v) gelatin, 200 µM dATP, 200 µM dGTP, 200 µM dGTP, 200 µM dTTP, 1.0 µM each primer, 100 ng/ 100 ul genomic DNA as template or 50 ng/100 µl plasmid DNA as template. The reactions were overlaid with mineral oil and heated to 95°C for 5 minutes. 2.5 Units/100 µl *AmpliTaq* (trademark) polymerase (Perkin Elmer Cetus) was added and the cycling was started. The samples were typically incubated 94°C for 1 minute and 30 seconds, 37-50°C for 2 minutes, 72°C for 3 minutes for 25-30 cycles. This was followed by a 7 minute at 72°C incubation then stored at 4°C.

Fragment A (SEQ ID NO:12) consisted of nucleotides 5-95 of MK14; fragment B (SEQ ID NO:13) consisted of nucleotides 96-202 of MK14 (note comment in Example 4 above); fragment C (SEQ ID NO:14) consisted of nucleotides 203-337 of MK14; fragment D (SEQ ID NO:15) consisted of nucleotides 338-429 of MK14; and fragment E (SEQ ID NO:16) consisted of nucleotides 430-527 of MK14.

### EXAMPLE 6

### Hybridisation Patterns of MK14 Fragments A-E as Determined by Genomic Southern Blot Analysis

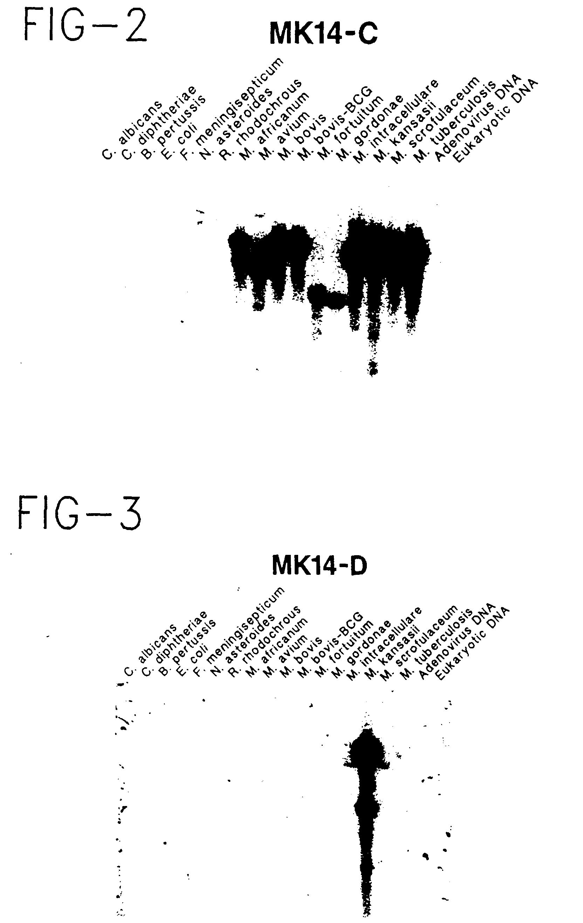

MK14 fragments A-E produced in Example 5 above were used as probes for Southern blot analysis of genomic DNA from a variety of mycobacteria and non-mycobacteria.
All fragments produced unique hybridization patterns. Fragment C (MK14-C)(SEQ ID NO:14) showed strong genus specificity **(figure 2**), whereas fragment D (MK14-D)(SEQ ID NO:15) showed strong *M. kansasii* specificity (**figure 3**). Fragment A (MK14-A)(SEQ ID NO:12) hybridized to all species of Mycobacteria tested, but cross reacted to a few nonmycobacterial species. Fragment B (MK14-B (SEQ ID No:13) hybridized strongly t*o M. kansasii*, hybridized weakly to TB complex organisms and *M. gordonae*, and showed no cross-reactivity to the non-mycobacterial species tested. Fragment E (MK14-E)(SEQ ID NO:16) hybridized well to *M*. *bovis, M. gordonae, M. intracellulare,* and *M. kansasii*, did not hybridize to the other mycobacterial species tested, and showed no cross-reactivity to the non-mycobacterial species tested.

For Southern blot analysis, genomic DNA was digested with *PstI* (GIBCO BRL). The reactions were separated on a 0.8% agarose gel then transferred to a nylon membrane (GIBCO BRL nylon-1 or NEN-DuPont Gene Screen). *See* J. Sambrook et al., Molecular Cloning, A Laboratory Manual (2d Ed. 1989)(Cold Spring Harbor Laboratory). Filters were then UV cross-linked (Stratalinker) and hybridized (GIBCO BRL) (Sambrook et. al.). The probes were labeled by random primers (Boehringer Mannheim Biochemicals) using [α-³²P]-deoxyadenosine triphosphate and [α-³²P]-deoxycytidine triphosphate (NEN-DuPont). The blots were washed at -25°C to -10°C below the predicted Tm. (Sambrook et. al.). The filters were exposed to XAR-5 film for 1-7 days.

### EXAMPLE 7

### Hybridization Patterns of MK14 Fragments C1 and C2 as Determined by Genomic Southern Blot Analysis

Fragment MK14-C was divided into two smaller fragments by PCR amplification as described above with oligonucleotides MK14-13 (TAG TAT CGA CTG CGT)(SEQ ID NO:17) and MK 14-14 (TCT TGC CCG TTG GGG)(SEQ ID NO:18), and with oligonucleotides MK14-15 (CCC CAA CGG GCA AGA) (SEQ ID NO:19) and MK14-10 (SEQ ID NO:9) to produce fragment MK14-C1 (SEQ ID NO:20) and fragment MK14-C2 (SEQ ID NO:21) respectively. MK14-C1 hybridized to all the slow growing mycobacteria, however it failed to hybridize to *M. fortuitum* and *M. gordonae.* Fragment MK14-C2 did hybridize to all mycobacteria tested and did not cross hybridize to the non-mycobacterial DNA's tested.

### EXAMPLE 8

### PCR Analysis of Fragment MK14-C

MK14-C was analyzed further by PCR analysis. A PCR amplification reaction containing oligos 14-3 (SEQ ID NO:2) and 14-10 (SEQ ID NO:9), using many mycobacterial and non-mycobacterial DNA's as templates, showed the best genus specificity (**figure 4**). These oligos amplify a 242 bp product containing fragments B and C (figure 1)(SEQ ID NO:22). There was a product of the correct size in all of the slow growing mycobacteria tested. The fast growing mycobacteria, *M. chelonae, M. fortuitum* and *M. gordonae,* did not show a 242 bp band on an ethidium bromide stained agarose gel. A specific probe to an internal region of the amplified product will show only specific products which may not be visualized on an ethidium bromide stained agarose gel. It will also prove that the 242 bp product seen in the slow growing mycobacteria is in fact the specific MK14-C-like product.

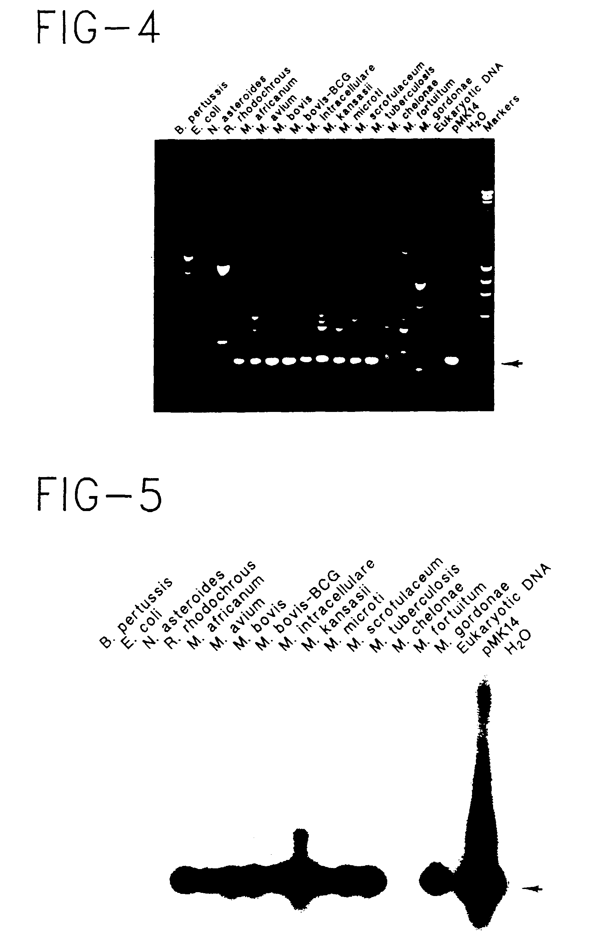

A southern blot was done on these PCR amplification reactions using MK14-C as the probe. PCR Southern blots were done in the same manner as the genomic southern blots described above, except 5 µl of the PCR reaction was separated on a 1.0% agarose gel. The probe hybridized to all the slow growing mycobacteria and *M. gordonae.* It did not hybridize to *M. chelonae, M*. *fortuitum, E. coli, B. pertussis, N. asteroides, R. rhodochrous* or eukaryotic DNA (**figure 5**).

### EXAMPLE 9

### Subcloning MK14 fragments B and C from M. avium and M. tuberculosis

Choosing oligonucleotides that will amplify all mycobacteria is dependent on how homologous the MK14-C DNA sequence is among different species. Therefore, it was decided to sequence the analogous DNA from two additional species, *M. avium* and *M. tuberculosis.* A comparison of the new sequences to the *M. kansasii* sequence could then be used to design specific oligonucleotides.

PCR amplification reactions were carried out using oligos 14-3 (SEQ ID NO:2) and 14-10 (SEQ ID NO:9) with *M. avium*, *M. tuberculosis* and *M. kansasii* genomic DNA as templates. The 242 bp product was isolated by Low Melting Point agarose (GIBCO BRL). They were then kinased in 50 mM Tris-HCl pH 8.8, 10 mM MgCl₂, 5% glycerol, 5 mM DTT, .5 mM ATP and 10 Units/20 µl Polynucleotide Kinase (Stratagene) for 30 minutes at 37°C. The vector, BlueScript SK+ (trademark), was digested with *HincII* (GIBCO BRL) then phosphatased with CIAP (Boehringer Mannheim Biochemicals). The insert and vector were ligated in a 3 to 1 ratio overnight at 15°C. Subclones of each were sequenced as described above.

Comparing *M. kansasii* fragment BC (SEQ ID NO:22) with *M. tuberculosis* fragment BC (SEQ ID NO:23) or *M. avium* fragment BC (SEQ ID NO:24), the homologies are 79% and 80% respectively. Even when *M. avium* is compared to *M. tuberculo*sis the homology is 76%. When all three are aligned, the homology is 69%.

### EXAMPLE 10

### Screening of PCR Amplification Reactions by Primer Extension with Oligos 14-23 and 14-15

The same set of PCR amplification reactions were screened for specificity by primer extension using oligo 14-23 (figure 4). Oligo 14-23 (CTA GTG AAT GGG A) (SEQ ID NO:25) was designed using the sequence homology information described above. The results showed the same specificity as the Southern blots (data not shown). The varying intensities of the bands can be explained by the oligo sequence. The oligo sequence is identical in *M. tuberculosis* and *M. kansasii*, however there is one mismatch in *M. avium.*

For primer extension, 2 pmoles of oligonucleotide 14-23 was kinased in 1X REact 1 (BRL) with 4.6 pmoles of [g-³²P]-adenosine triphosphate (NEN-DuPont) and 1 unit/µl Polynucleotide Kinase (Stratagene). The reaction was incubated at 37°C for 30 minutes then heated to 65°C for 10 minutes. The reaction was then diluted to 2X the volume with TE (10 mM Tris/8.0, 1 mM EDTA). For the primer extension reaction 5 µl of a PCR reaction was diluted to 10 µl in 1X PCR buffer (Perkin Elmer Cetus), .125 mM dNTP's, and .05 pmoles/10 µl of labeled primer. The reaction was heated to 95°C for 10 minutes then cooled to room temperature. 1.5 units of Klenow Large Fragment (BRL) was added and the samples were incubated at 37°C for 10 minutes. Stop solution (USB) was added and the samples were heated to 95°C for 5 minutes before separating on an 8% acrylamide denaturing gel. Gels were then exposed to XAR film for 2-16 hours.

Another primer extension reaction was done on the same PCR amplified products using oligo 14-15 (SEQ ID NO:16). This oligo has very little homology between *M. kansasii, M. tuberculosis* and *M. avium* with 6-7 mismatches in 13 bp. The only products detected were those from *M. kansasii* and the control plasmid, pMK14 (data not shown). There was also a slight band seen in *M. gordonae*. Using two different oligos to detect the same PCR amplification reaction by primer extension, gave dramatically different profiles. Oligo 14-23 showed genus specificity, whereas oligo 14-15 showed *M. kansasii* specificity. This shows the sequences provided here can be used for genus, *M. kansasii, M. tuberculosis* o*r M. avium* specificity depending on the assay and detection methods used. In both the Southern blot and primer extension assays*, N. asteroides* and *M.* *fortuitum* showed very weak hybridisation after longer exposures.

The foregoing examples are illustrative of the present invention, and are not to be construed as limiting thereof. The invention is defined by the following claims.

## Claims

1. An oligonucleotide probe capable of selectively hybridising to Mycobacteria nucleic acid, said oligonucleotide probe selected from the group consisting of:
(a) an oligonucleotide probe consisting of the DNA sequence given herein as SEQ ID NO:1 (MK14):
(b) oligonucleotide probes which hybridise to MK 14, are at least 10 nucleotides in length, and are at least 60% homologous with MK14 nucleic acid; and
(c) oligonucleotide probes which are complementary to any of the foregoing and are capable of selectively hybridising to Mycobacteria nucleic acid.

2. An oligonucleotide probe according to claim 1, consisting of the sequences given herein as SEQ ID NO:1 (MK14):

3. An oligonucleotide probe according to claim 1, selected from the group consisting of probes consisting of the sequences given herein as SEQ ID NO:12 (MK14-A): SEQ ID NO:13 (MK14-B): SEQ ID NO:14 (MK14-C): SEQ ID NO:15 (MK14-D): SEQ ID NO:16 (MK14-E): SEQ ID NO:20 (MK14-C1): and SEQ ID NO:21 (MK14-C2):

4. An oligonucleotide probe according to claim 1, selected from the group consisting of:
(a) an oligonucleotide probe consisting of the DNA sequence given herein as SEQ ID NO: 22 (M.Kansasii fragment BC): an oligonucleotide probe consisting of the DNA sequence given herein as SEQ ID NO:23 (M.tuberculosis fragment BC): and an oligonucleotide probe consisting of the sequence given herein as SEQ ID NO:24 (M.avium fragment BC):
(b) oligonucleotide probes which hybridise to probes of (a) above, are at least 10 nucleotides in length, and are at least 60% homologous with MK14 nucleic acid; and
(c) oligonucleotide probes which are complementary to any of the foregoing and are capable of selectively hybridising to Mycobacteria nucleic acid.

5. A method of detecting Mycobacteria nucleic acid in a nucleic acid sample, comprising:
(a) contacting an oligonucleotide probe to a nucleic acid sample under conditions permitting the hybridisation of said oligonucleotide probe to Mycobacteria nucleic acid, wherein said oligonucleotide probe is selected from the group consisting of
(i) an oligonucleotide probe consisting of the DNA sequence given herein as SEQ ID NO:1 (MK14):
(ii) oligonucleotide probes which hybridise to MK14, are at least 10 nucleotides in length, and are at least 60% homologous with MK14 nucleic acid; and
(iii) oligonucleotide probes which are complementary to any of the foregoing and are capable of selectively hybridising to Mycobacteria nucleic acid; and then
(b) detecting whether or not said oligonucleotide probe hybridises to said nucleic acid sample, the hybridisation of the oligonucleotide probe to the nucleic acid sample indicating that the nucleic acid sample contains Mycobacteria nucleic acid.

6. A method according to claim 5, further comprising a strand displacement amplification step preceding said detecting step.

7. A method of amplifying a selected Mycobacteria nucleic acid sequence in a nucleic acid sample, comprising:
(a) contacting at least one pair of oligonucleotide probes to the nucleic acid sample under conditions permitting the hybridisation of each of said oligonucleotide probes to the selected Mycobacteria nucleic acid sequence, wherein said pair of probes together comprise an amplification pair, and wherein each of said oligonucleotide probes is selected from the group consisting of
(i) oligonucleotide probes comprising fragments of SEQ ID NO:1 (MK14) which retain the capability of MK14 of selectively hybridising to Mycobacteria nucleic acid:
(ii) oligonucleotide probes which are complementary to any of the foregoing, are at least 10 nucleotides in length, and are at least 60% homologous with MK14 nucleic acid; and
(b) amplifying said selected Mycobacteria target sequence by cyclically reacting said at least one pair of oligonucleotide probes with said nucleic acid sample to produce an amplification product.

8. A method according to claim 7, wherein said amplifying step is carried out by strand displacement amplification.

9. A kit for detecting Mycobacteria nucleic acid in a nucleic acid sample, comprising a hybridisation solution together with an oligonucleotide probe selected from the group consisting of
(i) an oligonucleotide probe consisting of the DNA sequence given herein as SEQ ID NO:1 (MK14):
(ii) oligonucleotide probes which hybridise to MK14, are at least 10 nucleotides in length, are at least 60% homologous with MK14 nucleic acid; and
(iii) oligonucleotide probes which are complementary to any of the foregoing and are capable of selectively hybridising to Mycobacteria nucleic acid.

10. A kit according to claim 9, said oligonucleotide probe selected from the group consisting of oligonucleotide probes consisting of the sequences given herein as SEQ ID NO:12 (MK14-A): SEQ ID NO:13 (MK14-B): SEQ ID NO:14 (MK14-C): SEQ ID NO:15 (MK14-D): SEQ ID NO:16 (MK14-E): SEQ ID NO:20 (MK14-C1): and SEQ ID NO:21 (MK14-C2):

## Patentansprüche

1. Eine Oligonukleotid-Sonde, die zur selektiven Hybridisierung mit Mycobacterien-Nukleinsäure befähigt ist, wobei diese Oligonukleotid-Sonde aus der Gruppe ausgewählt ist, die besteht aus:
(a) einer Oligonukleotid-Sonde, die aus der hierin als SEQ ID NO:1 (MK14) angegebenen DNA Sequenz besteht:
(b) Oligonukleotid-Sonden, die mit MK14 hybridisieren, eine Länge von mindestens 10 Nukleotiden aufweisen und mindestens zu 60 % homolog zu MK14 Nucleinsäure sind; und
(c) Oligonukleotid-Sonden, die komplementär zu irgendeiner der vorhergehenden und imstande sind, selektiv mit Mycobakterien-Nucleinsäure zu hybridisieren.

2. Eine Oligonukleotid-Sonde nach Anspruch 1, die aus den hierin als SEQ ID NO:1 (MK14) angegebenen Sequenzen besteht:

3. Eine Oligonukleotid-Sonde nach Anspruch 1, die aus der Gruppe ausgewählt ist, die aus Sonden besteht, die aus den hierin als folgende angegebenen Sequenzen bestehen:
SEQ ID NO:12 (MK14-A) SEQ ID NO:13 (MK14-B): SEQ ID NO:14 (MK14-C): SEQ ID NO:15 (MK14-D): SEQ ID NO:16 (MK14-E): SEQ ID NO:20 (MK14-C1): and SEQ ID NO:21 (MK14-C2):

4. Eine Oligonukleotid-Sonde nach Anspruch 1, die aus der Gruppe ausgewählt ist, die besteht aus:
(a) einer Oligonukleotid-Sonde, die aus der hierin als SEQ ID NO: 22 (M. Kansasii Fragment BC) angegebenen DNA-Sequenz besteht: einer Oligonukleotid-Sonde, die aus der hierin als SEQ ID NO: 23 (M. tuberculosis Fragment BC) angegebenen DNA-Sequenz besteht: und einer Oligonukleotid-Sonde, die aus der hierin als SEQ ID NO: 24 (M. avium Fragment BC) angegebenen DNA-Sequenz besteht:
(b) Oligonukleotid-Sonden, die mit obigen Sonden von (a) hybridisieren, eine Länge von mindestens 10 Nukleotiden aufweisen und mindestens zu 60 % homolog zu MK14 Nucleinsäure sind ; und
(c) Oligonukleotid-Sonden, die komplementär zu irgendeiner der vorhergehenden und imstande sind, selektiv mit Mycobakterien-Nucleinsäure zu hybridisieren.

5. Ein Verfahren zur Feststellung von Mycobakterien-Nukleinsäure in einer Nukleinsäure-Probe, welches Verfahren umfaßt:
(a) In-Kontakt-Bringen einer Oligonukleotid-Sonde mit einer Nukleinsäure-Probe unter Bedingungen, die die Hybridisierung der genannten Oligonukleotid-Sonde mit Mycobakterien-Nukleinsäure erlaubt, wobei die genannte Oligonukleotid-Sonde ausgewählt ist aus der Gruppe, die besteht aus:
(i) einer Oligonukleotid-Sonde, die aus der hierin als SEQ ID NO: 1 (MK14) angegebenen DNA-Sequenz besteht:
(ii) Oligonukleotid-Sonden, die mit MK14 hybridisieren, eine Länge von mindestens 10 Nukleotiden aufweisen und mindestens zu 60 % homolog zu MK14 Nukleinsäure sind ; und
(iii) Oligonukleotid-Sonden, die komplementär zu irgendeiner der vorhergehenden und imstande sind, selektiv mit Mycobakterien-Nukleinsäure zu hybridisieren; und dann
(b) Festellen, ob die genannte Oligonukleotid-Sonde mit der genannten Nukleinsäure-Probe hybridisiert oder nicht, wobei die Hybridisierung der Oligonukleotid-Sonde mit der Nukleinsäure-Probe darauf hindeutet, daß die Nukleinsäure-Probe Mycobakterien-Nukleinsäure enthält.

6. Ein Verfahren nach Anspruch 5, das weiters einen Schritt der Strangverschiebungsamplifizierung umfaßt, welcher vor dem genannten Feststellungsschritt erfolgt.

7. Ein Verfahren zur Amplifizierung einer ausgewählten Mycobakterien-Nukleinsäuresequenz in einer Nukleinsäure-Probe, welches Verfahren umfaßt:
(a) In-Kontakt-Bringen mindestens eines Paares von Oligonukleotid-Sonden mit der Nukleinsäure-Probe unter Bedingungen, die die Hybridisierung jeder der genannten Oligonukleotid-Sonden mit der ausgewählten Mycobakterien-Nukleinsäuresequenz gestatten, wobei das genannte Sondenpaar gemeinsam ein Amplifizierungspaar umfaßt und wobei jede der genannten Oligonukleotid-Sonden aus der Gruppe ausgewählt ist, die besteht aus:
(i) Oligonukleotid-Sonden, die Fragmente von SEQ ID NO:1 (MK14) umfassen, welche die Fähigkeit von MK14 zur selektiven Hybridisierung mit Mycobakterien-Nukleinsäure behalten:
(ii) Oligonukleotid-Sonden, die komplementär zu irgendeiner der vorhergehenden sind, eine Länge von mindestens 10 Nukleotiden aufweisen und mindestens zu 60 % homolog zu MK14 Nukleinsäure sind; und
(b) Amplifizierung der genannten ausgewählten Mycobakterien-Zielsequenz durch zyklische Umsetzung des genannten mindestens einen Paares von Oligonukleotid-Sonden mit der genannten Nukleinsäure-Probe zur Herstellung eines Amplifizierungsproduktes.

8. Ein Verfahren nach Anspruch 7, in welchem der genannte Amplifizierungsschritt durch Strangverschiebungsamplifizierung erfolgt.

9. Ein Kit zur Feststellung von Mycobakterien-Nukleinsäure in einer Nukleinsäure-Probe, der eine Hybridisierungslösung gemeinsam mit einer Oligonukleotid-Sonde umfaßt, welch letztere aus der Gruppe ausgewählt ist, die besteht aus:
(i) einer Oligonukleotid-Sonde, die aus der hierin als SEQ ID NO: 1 (MK14) angegebenen DNA-Sequenz besteht:
(ii) Oligonukleotid-Sonden, die mit MK14 hybridisieren, eine Länge von mindestens 10 Nukleotiden aufweisen und mindestens zu 60 % homolog zu MK14 Nukleinsäure sind; und
(iii) Oligonukleotid-Sonden, die komplementär zu irgendeiner der vorhergehenden und imstande sind, selektiv mit Mycobakterien-Nukleinsäure zu hybridisieren.

10. Ein Kit nach Anspruch 9, wobei die genannte Oligonukleotid-Sonde aus der Gruppe ausgewählt ist, die aus Oligonukleotid-Sonden besteht, die aus den hierin als folgende angegebenen Sequenzen bestehen:
SEQ ID NO:12 (MK14-A) SEQ ID NO:13 (MK14-B) SEQ ID NO:14 (MK14-C) SEQ ID NO:15 (MK14-D) SEQ ID NO:16 (MK14-E) SEQ ID NO:20 (MK14-C1) und SEQ ID NO:21 (MK14-C2)

## Revendications

1. Sonde oligonucléotidique capable de s'hybrider sélectivement avec de l'acide nucléique de mycobactéries, ladite sonde oligonucléotidique étant choisie parmi :
(a) une sonde oligonucléotidique consistant en la séquence d'ADN donnée ici sous la désignation ID SEQ n°: 1 (MK14) :
(b) des sondes oligonucléotidiques qui s'hybrident avec MK14, ont une longueur d'au moins 10 nucléotides et présentent une homologie d'au moins 60 % avec l'acide nucléique de MK14; et
(c) des sondes oligonucléotidiques qui sont complémentaires de l'une quelconque des précédentes, et sont capables de s'hybrider sélectivement avec de l'acide nucléique de mycobactéries.

2. Sonde oligonucléotidique selon la revendication 1, consistant en la séquence donnée ici sous la désignation ID SEQ n°: 1 (MK14):

3. Sonde oligonucléotidique selon la revendication 1, choisie parmi les sondes consistant en les séquences données ici sous les désignations
ID SEQ n°: 12 (MK14-A): ID SEQ n°: 13 (MK14-B): ID SEQ n°: 14 (MK14-C): ID SEQ n°: 15 (MK14-D): ID SEQ n°: 16 (MK14-E): ID SEQ n°: 20 (MK14-C1): et ID SEQ n°: 21 (MK14-C2):

4. Sonde oligonucléotidique selon la revendication 1, choisie parmi:
(a) une sonde oligonucléotidique consistant en la séquence d'ADN donnée ici sous la désignation ID SEQ n°: 22 (fragment BC de *M. kansasii*) : une sonde oligonucléotidique consistant en la séquence d'ADN donnée ici sous la désignation ID SEQ n°: 23 (fragment BC de *M. tuberculosis*) : et une sonde oligonucléotidique consistant en la séquence donnée ici sous la désignation ID SEQ n°: 24 (fragment BC de *M. avium*) :
(b) des sondes oligonucléotidiques qui s'hybrident avec les sondes de (a) ci-dessus, ont une longueur d'au moins 10 nucléotides et présentent une homologie d'au moins 60 % avec l'acide nucléique de MK14; et
(c) des sondes oligonucléotidiques qui sont complémentaires de l'une quelconque des précédentes et sont capables de s'hybrider sélectivement avec de l'acide nucléique de mycobactéries.

5. Procédé de détection d'acide nucléique de mycobactéries dans un échantillon d'acide nucléique, comprenant:
(a) la mise en contact d'une sonde oligonucléotidique avec un échantillon d'acide nucléique, dans des conditions permettant l'hybridation de ladite sonde oligonucléotidique avec de l'acide nucléique de mycobactéries, ladite sonde oligonucléotidique étant choisie parmi
(i) une sonde oligonucléotidique consistant en la séquence d'ADN donnée ici sous la désignation ID SEQ n°: 1 (MK14) :
(ii) des sondes oligonucléotidiques qui s'hybrident avec MK14, ont une longueur d'au moins 10 nucléotides et présentent une homologie d'au moins 60 % avec l'acide nucléique de MK14; et
(iii) des sondes oligonucléotidiques qui sont complémentaires de l'une quelconque des précédentes et sont capables de s'hybrider sélectivement avec de l'acide nucléique de mycobactéries et ensuite
(b) le fait de détecter si ladite sonde oligonucléotidique s'hybride ou non avec ledit échantillon d'acide nucléique, l'hybridation de la sonde oligonucléotidique avec l'échantillon d'acide nucléique indiquant que l'échantillon d'acide nucléique contient de l'acide nucléique de mycobactéries.

6. Procédé selon la revendication 5, comprenant en outre une étape d'amplification par déplacement de brin, précédant ladite étape de détection.

7. Procédé d'amplification d'une séquence choisie d'acide nucléique de mycobactéries, dans un échantillon d'acide nucléique, comprenant:
(a) la mise en contact d'au moins une paire de sondes oligonucléotidiques avec l'échantillon d'acide nucléique, dans des conditions permettant l'hybridation de chacune desdites sondes oligonucléotidiques avec la séquence choisie d'acide nucléique de mycobactéries, les deux sondes de ladite paire comprenant ensemble une paire d'amplification, et chacune desdites sondes oligonucléotidiques étant choisie parmi
(i) des sondes oligonucléotidiques comprenant des fragments d'ID SEQ n°: 1 (MK14) conservant l'aptitude de MK14 à s'hybrider sélectivement avec de l'acide nucléique de mycobactéries:
(ii) des sondes oligonucléotidiques qui sont complémentaires de l'une quelconque des précédentes, ont une longueur d'au moins 10 nucléotides et présentent une homologie d'au moins 60 % avec l'acide nucléique de MK14; et
(b) l'amplification de ladite séquence cible choisie de mycobactéries, par mise en réaction cyclique de ladite au moins une paire de sondes oligonucléotidiques avec ledit échantillon d'acide nucléique, pour la formation d'un produit d'amplification.

8. Procédé selon la revendication 7, dans lequel ladite étape d'amplification est effectuée par amplification par déplacement de brin.

9. Nécessaire pour la détection d'acide nucléique de mycobactéries dans un échantillon d'acide nucléique, comprenant une solution d'hybridation conjointement avec une sonde oligonucléotidique choisie parmi
(i) une sonde oligonucléotidique consistant en la séquence d'ADN donnée ici sous la désignation ID SEQ n°: 1 (MK14) :
(ii) des sondes oligonucléotidiques qui s'hybrident avec MK14, ont une longueur d'au moins 10 nucléotides et présentent une homologie d'au moins 60 % avec l'acide nucléique de MK14; et
(iii) des sondes oligonucléotidiques qui sont complémentaires de l'une quelconque des précédentes et sont capables de s'hybrider sélectivement avec de l'acide nucléique de mycobactéries.

10. Nécessaire selon la revendication 9, ladite sonde oligonucléotidique étant choisie parmi les sondes oligonucléotidiques consistant en les séquences données ici sous les désignations
ID SEQ n°: 12 (MK14-A) : ID SEQ n°: 13 (MK14-B) : ID SEQ n°: 14 (MK14-C) : ID SEQ n°: 15 (MK14-D) : ID SEQ n°: 16 (MK14-E) : ID SEQ n°: 20 (MK14-C1) : et ID SEQ n°: 21 (MK14-C2) :


### SEQUENCE LISTING

### (1) GENERAL INFORMATION:

(i) APPLICANT: BECTON, DICKINSON AND COMPANY
   (A) NAME: BECTON, DICKINSON AND COMPANY
   (B) STREET: 1 Becton Drive
   (C) CITY: Franklin Lakes
   (D) STATE: New Jersey
   (E) COUNTRY: USA
   (F) ZIP: 07417-1880
(ii) TITLE OF INVENTION: Mycobacteria Probes
(iii) NUMBER OF SEQUENCES: 25
(iv) COMPUTER READABLE FORM:
   (A) MEDIUM TYPE: Floppy disk
   (B) COMPUTER: IBM PC compatible
   (C) OPERATING SYSTEM: PC-DOS/MS-DOS
   (D) SOFTWARE: PatentIn Release #1.0, Version #1.25
(v) CURRENT APPLICATION DATA:
   (A) APPLICATION NUMBER: 93108325.7
   (B) FILING DATE: May 24, 1993

### (2) INFORMATION FOR SEQ ID NO:1:

(i) SEQUENCE CHARACTERISTICS:
   (A) LENGTH: 531 base pairs
   (B) TYPE: nucleic acid
   (C) STRANDEDNESS: single
   (D) TOPOLOGY: linear
(ii) MOLECULE TYPE: cDNA
(xi) SEQUENCE DESCRIPTION: SEQ ID NO:1:
   GATCGGCTCC GGCGAACGAT TCCGCCAGGG ACGGCCGGCG CACCCCGGCC GCCGGCGTGC    60
   TCACCTCAAG CCTCGGCGGC GAACCCGACG CGTCGACTCT GTGTGCCACC ACACCATCAT   120
   CCGCCATGGC CGCGACCAGT TAGCCAGTTA GTGTGACGGC ACTCTTCCCG GGCTTCACCC   180
   ACAGGTAGCC GATGAGCCGT ATGAGCCCCC GCACGCCGTA GTATCGACTG CGTCTAGTGA   240
   ATGGGAATCG GCGGAGAGTG ACAGAGCATA TCCCGGCCCC AACGGGCAAG ACGGATGGGC   300
   GCAAGCGGCG CTGGCACCAG CACAAGGTGG AGCGGCGCAA CGAACTCGTT GACGGGACAA   360
   TCGAGGCCAT ACGTGTCCGC GGCCGCTTCC TGAGTATGGA CGAGATCCGT GAAGAAAACC   420
   CGCAGGAATG GGCGCGCATG GCTCAGGGGC AGCTGCCCAG CGCCGTCGAC GAGGATGCCT   480
   TCCGCGCGCG GGTCCGCGCG GCTGTCGACC ACGTGGTCGT CAGCGCCGAT C            531

### (2) INFORMATION FOR SEQ ID NO:2:

(i) SEQUENCE CHARACTERISTICS:
   (A) LENGTH: 20 base pairs
   (B) TYPE: nucleic acid
   (C) STRANDEDNESS: single
   (D) TOPOLOGY: linear
(ii) MOLECULE TYPE: cDNA
(xi) SEQUENCE DESCRIPTION: SEQ ID NO:2:
   ACCTGTGTGC CACCACACCA    20

### (2) INFORMATION FOR SEQ ID NO:3:

(i) SEQUENCE CHARACTERISTICS:
   (A) LENGTH: 20 base pairs
   (B) TYPE: nucleic acid
   (C) STRANDEDNESS: single
   (D) TOPOLOGY: linear
(ii) MOLECULE TYPE: cDNA
(xi) SEQUENCE DESCRIPTION: SEQ ID NO:3:
   ATACGGTCAT CGGCTACCTG 20

### (2) INFORMATION FOR SEQ ID NO:4:

(i) SEQUENCE CHARACTERISTICS:
   (A) LENGTH: 20 base pairs
   (B) TYPE: nucleic acid
   (C) STRANDEDNESS: single
   (D) TOPOLOGY: linear
(ii) MOLECULE TYPE: cDNA
(xi) SEQUENCE DESCRIPTION: SEQ ID NO:4:
   AACGAACTCG TTGACGGGAC 20

### (2) INFORMATION FOR SEQ ID NO:5:

(i) SEQUENCE CHARACTERISTICS:
   (A) LENGTH: 20 base pairs
   (B) TYPE: nucleic acid
   (C) STRANDEDNESS: single
   (D) TOPOLOGY: linear
(ii) MOLECULE TYPE: cDNA
(xi) SEQUENCE DESCRIPTION: SEQ ID NO:5:
   ATTCCTGCGG GTTTTCTTCA 20

### (2) INFORMATION FOR SEQ ID NO:6:

(i) SEQUENCE CHARACTERISTICS:
   (A) LENGTH: 20 base pairs
   (B) TYPE: nucleic acid
   (C) STRANDEDNESS: single
   (D) TOPOLOGY: linear
(ii) MOLECULE TYPE: cDNA
(xi) SEQUENCE DESCRIPTION: SEQ ID NO:6:
   GGCTCCGGCG AACGATTCCG 20

### (2) INFORMATION FOR SEQ ID NO:7:

(i) SEQUENCE CHARACTERISTICS:
   (A) LENGTH: 20 base pairs
   (B) TYPE: nucleic acid
   (C) STRANDEDNESS: single
   (D) TOPOLOGY: linear
(ii) MOLECULE TYPE: cDNA
(xi) SEQUENCE DESCRIPTION: SEQ ID NO:7:
   CGACGCGTCG GGTTCGCCGC 20

### (2) INFORMATION FOR SEQ ID NO:8:

(i) SEQUENCE CHARACTERISTICS:
   (A) LENGTH: 20 base pairs
   (B) TYPE: nucleic acid
   (C) STRANDEDNESS: single
   (D) TOPOLOGY: linear
(ii) MOLECULE TYPE: cDNA
(xi) SEQUENCE DESCRIPTION: SEQ ID NO:8:
   GAGCCCCCGC ACGCCGTAGT 20

### (2) INFORMATION FOR SEQ ID NO:9:

(i) SEQUENCE CHARACTERISTICS:
   (A) LENGTH: 20 base pairs
   (B) TYPE: nucleic acid
   (C) STRANDEDNESS: single
   (D) TOPOLOGY: linear
(ii) MOLECULE TYPE: cDNA
(xi) SEQUENCE DESCRIPTION: SEQ ID NO:9:
   CGCCGCTCCA CCTTGTGCTG 20

### (2) INFORMATION FOR SEQ ID NO:10:

(i) SEQUENCE CHARACTERISTICS:
   (A) LENGTH: 20 base pairs
   (B) TYPE: nucleic acid
   (C) STRANDEDNESS: single
   (D) TOPOLOGY: linear
(ii) MOLECULE TYPE: cDNA
(xi) SEQUENCE DESCRIPTION: SEQ ID NO:10:
   GGGCGCGCAT GGCTCAGGGG 20

### (2) INFORMATION FOR SEQ ID NO:11:

(i) SEQUENCE CHARACTERISTICS:
   (A) LENGTH: 20 base pairs
   (B) TYPE: nucleic acid
   (C) STRANDEDNESS: single
   (D) TOPOLOGY: linear
(ii) MOLECULE TYPE: cDNA
(xi) SEQUENCE DESCRIPTION: SEQ ID NO:11:
   GGCGCTGACG ACCACGTGGT 20

### (2) INFORMATION FOR SEQ ID NO:12:

(i) SEQUENCE CHARACTERISTICS:
   (A) LENGTH: 91 base pairs
   (B) TYPE: nucleic acid
   (C) STRANDEDNESS: single
   (D) TOPOLOGY: linear
(ii) MOLECULE TYPE: cDNA
(xi) SEQUENCE DESCRIPTION: SEQ ID NO:12:
   GGCTCCGGCG AACGATTCCG CCAGGGACGG CCGGCGCACC CCGGCCGCCG GCGTGCTCAC 60
   CTCAAGCCTC GGCGGCGAAC CCGACGCGTC G 91

### (2) INFORMATION FOR SEQ ID NO:13:

(i) SEQUENCE CHARACTERISTICS:
   (A) LENGTH: 107 base pairs
   (B) TYPE: nucleic acid
   (C) STRANDEDNESS: single
   (D) TOPOLOGY: linear
(ii) MOLECULE TYPE: cDNA
(xi) SEQUENCE DESCRIPTION: SEQ ID NO:13:
   ACTCTGTGTG CCACCACACC ATCATCCGCC ATGGCCGCGA CCAGTTAGCC AGTTAGTGTG 60
   ACGGCACTCT TCCCGGGCTT CACCCACAGG TAGCCGATGA GCCGTAT 107

### (2) INFORMATION FOR SEQ ID NO:14:

(i) SEQUENCE CHARACTERISTICS:
   (A) LENGTH: 135 base pairs
   (B) TYPE: nucleic acid
   (C) STRANDEDNESS: single
   (D) TOPOLOGY: linear
(ii) MOLECULE TYPE: cDNA
(xi) SEQUENCE DESCRIPTION: SEQ ID NO:14:
   GAGCCCCCGC ACGCCGTAGT ATCGACTGCG TCTAGTGAAT GGGAATCGGC GGAGAGTGAC     60
   AGAGCATATC CCGGCCCCAA CGGGCAAGAC GGATGGGCGC AAGCGGCGCT GGCACCAGCA    120
   CAAGGTGGAG CGGCG                                                    135

### (2) INFORMATION FOR SEQ ID NO:15:

(i) SEQUENCE CHARACTERISTICS:
   (A) LENGTH: 92 base pairs
   (B) TYPE: nucleic acid
   (C) STRANDEDNESS: single
   (D) TOPOLOGY: linear
(ii) MOLECULE TYPE: cDNA
(xi) SEQUENCE DESCRIPTION: SEQ ID NO:15:
   CAACGAACTC GTTGACGGGA CAATCGAGGC CATACGTGTC CGCGGCCGCT TCCTGAGTAT     60
   GGACGAGATC CGTGAAGAAA ACCCGCAGGA AT                                  92

### (2) INFORMATION FOR SEQ ID NO:16:

(i) SEQUENCE CHARACTERISTICS:
   (A) LENGTH: 98 base pairs
   (B) TYPE: nucleic acid
   (C) STRANDEDNESS: single
   (D) TOPOLOGY: linear
(ii) MOLECULE TYPE: cDNA
(xi) SEQUENCE DESCRIPTION: SEQ ID NO:16:
   GGGCGCGCAT GGCTCAGGGG CAGCTGCCCA GCGCCGTCGA CGAGGATGCC TTCCGCGCGC     60
   GGGTCCGCGC GGCTGTCGAC CACGTGGTCG TCAGCGCC                            98

### (2) INFORMATION FOR SEQ ID NO:17:

(i) SEQUENCE CHARACTERISTICS:
   (A) LENGTH: 15 base pairs
   (B) TYPE: nucleic acid
   (C) STRANDEDNESS: single
   (D) TOPOLOGY: linear
(ii) MOLECULE TYPE: cDNA
(xi) SEQUENCE DESCRIPTION: SEQ ID NO:17:
   TAGTATCGAC TGCGT 15

### (2) INFORMATION FOR SEQ ID NO:18:

(i) SEQUENCE CHARACTERISTICS:
   (A) LENGTH: 15 base pairs
   (B) TYPE: nucleic acid
   (C) STRANDEDNESS: single
   (D) TOPOLOGY: linear
(ii) MOLECULE TYPE: cDNA
(xi) SEQUENCE DESCRIPTION: SEQ ID NO:18:
   TCTTGCCCGT TGGGG 15

### (2) INFORMATION FOR SEQ ID NO:19:

(i) SEQUENCE CHARACTERISTICS:
   (A) LENGTH: 15 base pairs
   (B) TYPE: nucleic acid
   (C) STRANDEDNESS: single
   (D) TOPOLOGY: linear
(ii) MOLECULE TYPE: cDNA
(xi) SEQUENCE DESCRIPTION: SEQ ID NO:19:
   CCCCAACGGG CAAGA 15

### (2) INFORMATION FOR SEQ ID NO:20:

(i) SEQUENCE CHACTERISTICS:
   (A) LENGTH: 73 base pairs
   (B) TYPE: nucleic acid
   (C) STRANDEDNESS: single
   (D) TOPOLOGY: linear
(ii) MOLECULE TYPE: cDNA
(xi) SEQUENCE DESCRIPTION: SEQ ID NO:20:
   TAGTATCGAC TGCGTCTAGT GAATGGGAAT CGGCGGAGAG TGACAGAGCA TATCCCGGCC 60
   CCAACGGGCA AGA 73

### (2) INFORMATION FOR SEQ ID NO:21:

(i) SEQUENCE CHARACTERISTICS:
   (A) LENGTH: 61 base pairs
   (B) TYPE: nucleic acid
   (C) STRANDEDNESS: single
   (D) TOPOLOGY: linear
(ii) MOLECULE TYPE: cDNA
(xi) SEQUENCE DESCRIPTION: SEQ ID NO:21:
   CCCCAACGGG CAAGACGGAT GGGCGCAAGC GGCGCTGGCA CCAGCACAAG GTGGAGCGGC     60
   G                                                                    61

### (2) INFORMATION FOR SEQ ID NO:22:

(i) SEQUENCE CHARACTERISTICS:
   (A) LENGTH: 241 base pairs
   (B) TYPE: nucleic acid
   (C) STRANDEDNESS: single
   (D) TOPOLOGY: linear
(ii) MOLECULE TYPE: cDNA
(xi) SEQUENCE DESCRIPTION: SEQ ID NO:22:
   ACCTGTGTGC CACCACACCA TCATCCGCCA TGGCCGCGAC CAGTTAGCCA GTTAGTGTGA     60
   CGGCACTCTT CCCGGGCTTC ACCCACAGGT AGCCGATGAG CCGTATGAGC CCCCGCACGC    120
   CGTAGTATCG ACTGCGTCTA GTGAATGGGA ATCGGCGGAG AGTGACAGAG CATATCCCGG    180
   CCCCAACGGG CAAGACGGAT GGGCGCAAGC GGCGCTGGCA CCAGCACAAG GTGGAGCGGC    240
   G                                                                   241

### (2) INFORMATION FOR SEQ ID NO:23:

(i) SEQUENCE CHARACTERISTICS:
   (A) LENGTH: 230 base pairs
   (B) TYPE: nucleic acid
   (C) STRANDEDNESS: single
   (D) TOPOLOGY: linear
(ii) MOLECULE TYPE: cDNA
(xi) SEQUENCE DESCRIPTION: SEQ ID NO:23:
   ACCTGTGTGC CACCACACCA TCATCCGCTA TCACCGGCAC CAGTTAATGT GACGACACTG 60
   TCCTGCGCAC CCCGCCGSCG CGTCCAATGT GCCAGCAGGA CCCCCAATTG GCCGTAGTAT 120
   CGGGAGCGTC TAGTGAATGG GAATTGGCAA CAGTGGCAGA GCGTATCCCG GCCGTGACCG 180
   TGAAGACGGA TGTGCAAGCG ACGCTGGCAC CAGCACAAGG TGGAGCGGCG 230

### (2) INFORMATION FOR SEQ ID NO:24:

(i) SEQUENCE CHARACTERISTICS:
   (A) LENGTH: 230 base pairs
   (B) TYPE: nucleic acid
   (C) STRANDEDNESS: single
   (D) TOPOLOGY: linear
(ii) MOLECULE TYPE: cDNA
(xi) SEQUENCE DESCRIPTION: SEQ ID NO:24:
   ACCTGTGTGC CACCACACCA TCATCTATST TTCCCGCCGA TTGGTGTGAC GACACCGCGC 60
   GCGACGGTCG TCCACGCCCG GGCCAAGACC GCGAACCAGC CGGTCCACGC CGTAGTATCG 120
   AGGGCGTCTA GGGAATGGGA TTTGGCGATA GTGGCAGAGC AAATCACGGC TGCGAGCGTC 180
   AAAATGGACG GGCGCAAGCG GCGCTGGCAT CAGCACAAGG TGGAGCGGCG 230

### (2) INFORMATION FOR SEQ ID NO:25:

(i) SEQUENCE CHARACTERISTICS:
   (A) LENGTH: 13 base pairs
   (B) TYPE: nucleic acid
   (C) STRANDEDNESS: single
   (D) TOPOLOGY: linear
(ii) MOLECULE TYPE: cDNA
(xi) SEQUENCE DESCRIPTION: SEQ ID NO:25:
   CTAGTGAATG GGA 13